# EUROPÄISCHE PATENTSCHRIFT

(11) **EP 0 439 796 B1**
(45) Veröffentlichungstag und Bekanntmachung des Hinweises auf die Patenterteilung: **24.01.1996**
(21) Anmeldenummer: 90125016.7
(22) Anmeldetag: 20.12.1990
(51) Int. Cl.: C07D 403/12, A61K 31/40

(54) **Indolylpropanole, Verfahren zu ihrer Herstellung und ihre Verwendung sowie die Verbindungen enthaltende Zubereitungen**
Indolylpropanols, process for their preparation, and their use and preparations containing them
Indolylpropanols, leur procédé de préparation, leur utilisation et préparations les contenant

(30) Priorität: 27.01.1990 DE 4002391
(43) Veröffentlichungstag der Anmeldung: 07.08.1991
(73) Patentinhaber: Beiersdorf-Lilly GmbH, D-20253 Hamburg (DE)
(72) Erfinder: Stenzel, Wolfgang, Dr., W-2057 Reinbek (DE); Armah, Ben, Dr., W-2000 Hamburg 52 (DE)
(74) Vertreter: Hudson, Christopher Mark

(56) Entgegenhaltungen:
- EP-A- 0 025 111
- EP-A- 0 297 380
- DE-A- 3 723 648
- FR-A- 2 567 885

## Beschreibung

### Indolylpropanole, Verfahren zu ihrer Herstellung und ihre Verwendung sowie die Verbindungen enthaltende Zubereitungen

Gegenstand der Erfindung sind neue substituierte Indolylpropanole der Formel I, 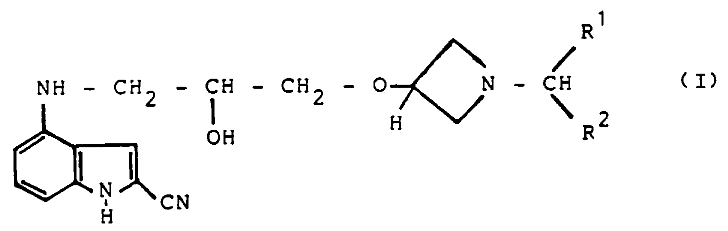 in der R¹ und R, die gleich oder verschieden sein können, jeweils Phenyl oder gegebenenfalls durch Halogen, Alkyl, C₃₋₇-Cycloalkyl oder C₁-₆-Alkoxy mono-oder gleich oder verschieden di-substituiertes Phenyl, einen 2-, 3- oder 4-Pyridinyl- oder einen 2- oder 3-Thienylrest bedeuten, wobei Alkyl geradkettiges Alkyl mit 1 bis 6 Kohlenstoffatomen oder verzweigtes Alkyl mit 3 bis 6 Kohlenstoffatomen bedeutet, sowie deren Salze und Säureadditionssalze, Tautomere und optische Isomere, Verfahren zu ihrer Herstellung, ihre Verwendung und Zubereitungen, die diese Verbindungen enthalten.

Der Einfachheit halber sind die erfindungsgemäßen Verbindungen in nur einer durch Formel I wiedergegebenen tautomeren Form definiert. Die Erfindung erstreckt sich -jedoch auf alle tautomeren Formen der Verbindungen.

Obgleich pharmazeutisch verträgliche Salze und Säureadditionssalze der neuen Verbindungen der Formel I bevorzugt sind, liegen alle Salze innerhalb des Bereichs der Erfindung. Alle Salze sind wertvoll zur Herstellung der Verbindung, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweisen, verwendet wird.

Die Verbindungen der allgemeinen Formel I und deren Salze enthalten asymmetrische Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Die Racemate können nach an sich bekannten Methoden in ihre optischen Antipoden aufgetrennt werden.

Mit den Verbindungen der vorliegenden Erfindung strukturell verwandten Verbindungen sind in den europäischen Patentanmeldungen 25 111 und 297 380 und den deutschen Offenlegungsschriften 35 24 955 und 37 23 648 beschrieben. Die Verbindungen der vorliegenden Erfindung werden aber von diesen Offenbarungen weder spezifisch offenbart noch nahegelegt.

Soweit nicht anders angegeben, können die erfindungsgemäßen Alkylgruppen und Alkylteile von Gruppen, z.B. Alkoxygruppen, geradkettig oder verzweigt sein und sie besitzen jeweils bevorzugt 1 bis 6 Kohlenstoffatome, vorzugsweise 1 bis 4 Kohlenstoffatome, insbesondere 1 oder 2 Kohlenstoffatome. Die verzweigten Alkylgruppen besitzen mindestens 3 Kohlenstoffatome. Bevorzugte Alkyle sind Methyl, Ethyl, n-Propyl, Isopropyl und Butyl. Besonders bevorzugt sind Methyl, Ethyl, Methoxy und Ethoxy.

Vorzugsweise besitzen erfindungsgemäße Cycloalkylgruppen 3 bis 7 Kohlenstoffatome, insbesondere 3 bis 6 Kohlenstoffatome. Besonders bevorzugt sind Cyclopropyl und Cyclohexyl.

Pyridinyl ist vorzugsweise Pyridin-4-yl, und Thienyl ist vorzugsweise Thien-3-yl. Geeignetes Halogen ist Fluor, Chlor, Brom oder Jod. Bevorzugt werden Fluor und Chlor.

Vorzugsweise sind R¹ und R unsubstituiertes Phenyl.

Die Phenylgruppe kann eine oder zwei der genannten Substituenten tragen, die gleich oder verschieden sein können. Sind die Phenylgruppen disubstituiert, dann sind die Substituenten vorzugsweise gleich.

Substituierte Phenylgruppen R¹ und/oder R sind mit den angegebenen Substituenten vorzugsweise in 3- und/oder 4-Stellung substituiert, insbesondere in 4-Stellung monosubstituiert. Bevorzugte Substituenten sind Halogen oder Alkoxy, insbesondere Halogen.

Die folgenden Verbindungen der Formel I, deren Salze und physiologisch verträgliche Salze werden bevorzugt:
a) R,S-4-(3-(1-Diphenylmethyl-azetidin-3-oxy)-2-hydroxypropylamino)-1H-indol-2-carbonitril
b) R,S-4-(3-(1-(Bis-4,4'-fluorphenylmethyl-azetidin-3-oxy)-2-hydroxypropylamino)-1H-indol-2-carbonitril
c) R,S-4-(3-(1-(4-Pyridinyl-phenyl-methyl-azetidin-3-oxy)-2-hydroxypropylamino)-1H-indol-2-carbonitril

Die neuen Verbindungen der allgemeinen Formel I, in der R¹ und R die angegebene Bedeutung haben, können hergestellt werden durch Umsetzung des Indolderivats II , 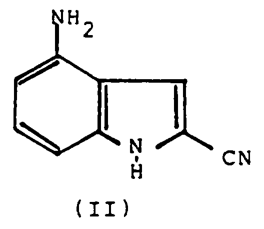 mit Verbindungen der Formel III, in der R¹ und R die obengenannte Bedeutung haben: 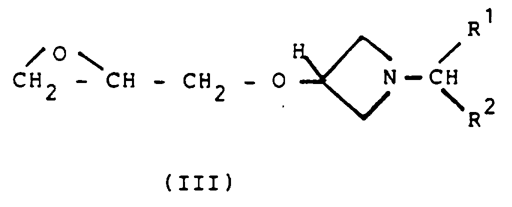 Die Umsetzungen können in organischen Lösungsmitteln, wie Ethanol oder Dioxan oder anderen geeigneten Lösungsmitteln in Gegenwart einer Base, vorzugsweise Piperidin, bei Temperaturen zwischen 0°C und der Siedetemperatur des Reaktionsgemisches, vorzugsweise bei Raumtemperatur, durchgeführt werden.

Die Verbindungen der Formeln II und III, in der R¹ und R die obengenannte Bedeutung haben, sind bekannt (deutsche Offenlegungsschrift 37 23 648 und europäische Patentanmeldung 297 380) oder können in Analogie zu bekannten Verfahren hergestellt werden.

Die Verbindung a) wird besonders bevorzugt.

Die erfindungsgemäßen Verbindungen der Formel I, ihre physiologisch verträglichen Salze und Säureadditionssalze sind therapeutische Wirkstoffe, besitzen hohe pharmakologische Wirkung und sind wertvolle Arzneimittel. Beispielsweise wirken sie als Natriumkanalmodulatoren. Insbesondere zeigen sie positiv inotrope und antiarrhythmische Wirkung. Sie eignen sich zur Behandlung von Herzinsuffizienz und Herzrhythmusstörungen.

Die Verbindungen der vorliegenden Erfindung können beim Menschen oral oder parenteral in einer Dosierung von 1-800 mg, vorzugsweise 10-200 mg, besonders bevorzugt 20-50 mg pro Tag, angewendet werden, insbesondere in unterteilten Dosen, zum Beispiel dreimal täglich. Diese Dosierungen sind vorteilhaft für die Behandlung der vorstehend genannten Krankheiten, insbesondere von Herzinsuffizienz und/oder Arrhythmien.

Die positiv inotrope Wirkung der erfindungsgemäßen Verbindungen wurde am Meerschweinchen-Papillarmuskel bestimmt (Naunyn-Schmiedeberg's Arch. Pharmacol. 304,37,1978). Die Konzentration der Substanz im Organbad betrug jeweils 10⁻⁵ mol/l. Die maximale prozentuale Steigerung der Kontraktionsamplitude wurde jeweils an drei Papillarmuskeln bestimmt und betrug mindestens 50%.

Gegenstand der Erfindung sind auch die erfindungsgemäßen Verbindungen zur Behandlung der vorstehenden Krankheiten sowie Verfahren zur Behandlung dieser Krankheiten, in denen diese Verbindungen verwendet werden sowie ihre Verwendung als Arzneimittel oder ihre Verwendung in Verfahren zur Herstellung von Mitteln, die diese Verbindungen enthalten, zur Behandlung dieser Krankheiten sowie Verfahren zur Herstellung der Verbindungen.

Gemäß der Erfindung werden pharmazeutische Zusammensetzungen geschaffen, die eine Verbindung der Formel I oder deren pharmazeutisch verträgliche Salze, gegebenenfalls zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Filmtabletten, Dragees, Sirups, Suspensionen und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tablette bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 1-30 mg.

Die Verbindungen der allgemeinen Formel I können sowohl Basen als auch Säuren beziehungsweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Schwefelsäure oder Halogenwasserstoffsäuren,zum Beispiel Salzsäure, und als organische Säuren zum Beispiel Fumarsäure, Maleinsäure, Zitronensäure und Weinsäure, geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, oder Kaliumhydroxid erhalten werden.

Die erfindungsgemäßen Verbindungen der Formel I weisen am Kohlenstoffatom 2 der Propoxyseitenkette ein Chiralitätszentrum auf und können, abhängig von den Substituenten, weitere asymmetrische Kohlenstoffatome besitzen und daher als Racemate und Diasteroisomere vorliegen. Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bildet, Trennung der Diastereoisomeren durch fraktionierte Kristallisation und Freisetzung der Enantiomeren durch geeignete Mittel. Besonders geeignete optisch aktive Säuren sind beispielsweise die d- und l-Formen der Weinsäure, Ditoluylweinsäure, Äpfelsäure, Mandelsäure, Kamphersulfonsäure oder Pyrrolidon-carbonsäure. Geeignete optisch aktive Basen sind alpha-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Gegenstand der Erfindung ist auch ein Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

Die folgenden Beispiele dienen zur Erläuterung der Erfindung.

### Beispiel 1

### (R,S)-4-(3-(1-Diphenylmethyl-azetidin-3-oxy)-2-hydroxypropylamino)-1H-indol-2-carbonitril

7,5 g Aminoindol-2-carbonitril und 16,1 g 1-(Diphenylmethyl)-3-(2,3-epoxypropoxy)-azetidin werden in 120 ml Ethanol in Gegenwart weniger Tropfen Piperidin 24 Stunden unter Rückfluß erhitzt und anschließend im Vakuum zur Trockne eingedampft. Das rohe Reaktionsgemisch wird in Diethylether verrührt, der unlösliche Rückstand abfiltriert und die Etherphase eingeengt. Die so vorgereinigte Verbindung wird säulenchromatographisch an Kieselgel gereinigt.
(CH₂Cl₂/Ethanol 99:1)
Ausbeute: 4,2 g (18%)
Schmp.: 88-89°C

### Beispiel 2

### Herstellung von Ampullen

Ampullen, die die im folgenden genannten Bestandteile enthalten, lassen sich in bekannter Weise herstellen. Der Wirkstoff wird in Wasser und 1,2-Propandiol gelöst und unter Stickstoff in Glasampullen abgefüllt.

(R,S)-4-(3-(1-Diphenylmethyl-azetidin-3-oxy)-2-hydroxypropylamino)-1H-indol-2-carbonitril 1 mg
1,2 Propandiol 0.8 ml
dest. Wasser ad 2.0 ml

### Beispiel 3

### Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Herzinsuffizienz in Dosierungsmengen von jeweils einer Tablette oder Kapsel dreimal täglich geeignet.

| Bestandteile | Gewicht (mg) | |
|---|---|---|
| | Tablette | Kapsel |
| (R,S)-4-(3-(1-Diphenylmethyl-azetidin-3-oxy)-2-hydroxypropylamino)-1H-indol-2-carbonitril | 10 | 5 |
| Tragacanth | 10 | |
| Lactose | 247,5 | 300 |
| Maisstärke | 25 | |
| Talk | 15 | |
| Magnesiumstearat | 2,5 | |

Analog zu den vorstehenden Beispielen können die in der folgenden Tabelle angegebenen erfindungsgemäßen Verbindungen der Formel I' erhalten werden. (Beispiele 4-13): 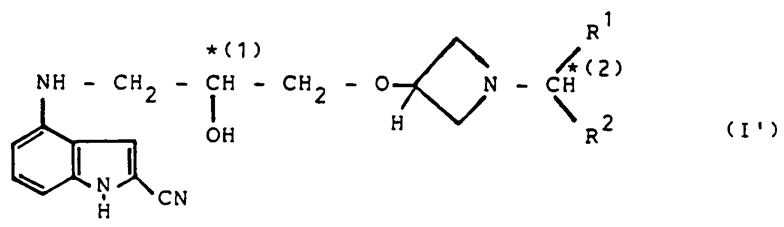 "rac" bedeutet racemisch.

"---" bedeutet, daß kein asymmetrisches Kohlenstoffatom vorliegt.

| Beispiel | R¹ | R | Konfig. C* (1) | Konfig. C* (2) |
|---|---|---|---|---|
| 4 | 4-F-Phenyl | 4-F-Phenyl | rac | --- |
| 5 | 4-OCH₃-Phenyl | 4-OCH₃-Phenyl | rac | --- |
| 6 | 4-CH₃-Phenyl | 4-CH₃-Phenyl | rac | --- |
| 7 | 4-Cl-Phenyl | Phenyl | rac | rac |
| 8 | 4-Pyridinyl | Phenyl | rac | rac |
| 9 | 3-Pyridinyl | Phenyl | rac | rac |
| 10 | 4-Pyridinyl | 4-Pyridinyl | rac | --- |
| 11 | 2-Thienyl | Phenyl | rac | rac |
| 12 | 3-Thienyl | Phenyl | rac | rac |
| 13 | 2-Thienyl | 2-Thienyl | rac | --- |

### Beispiel 14

### 1-(Diphenylmethyl-3-(2,3-epoxypropoxy)-azetidin

37,5 g Diphenylmethyl-azetidin-3-ol werden bei Raumtemperatur in einem Gemisch aus 250 ml Dimethylsulfoxid und 150 ml 5%iger Natronlauge gelöst, mit 65 ml Epichlorhydrin versetzt und 3 Tage bei Raumtemperatur stehen gelassen. Das Reaktionsgemisch wird mit 300 ml Methylenchlorid extrahiert, die organische Phase über Natriumsulfat getrocknet und eingedampft. Das Rohprodukt wird im Vakuum destilliert. Ausbeute: 29.0 g 1-(Diphenylmethyl)-3-(2,3-epoxypropoxy)-azetidin Kp. 174 - 176°C, 0,2 mbar

## Patentansprüche (Patentansprüche für folgende(n) Vertragsstaat(en): AT, BE, CH, DE, DK, FR, GB, IT, LI, NL, SE)

1. Substituierte Indolylpropanole der Formel I, 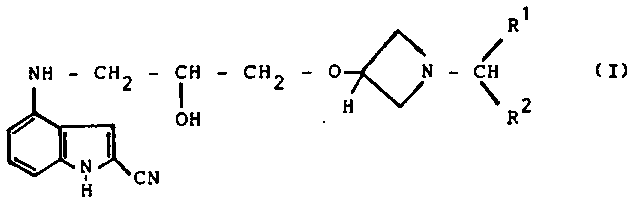 in der R¹ und R, die gleich oder verschieden sein können, jeweils Phenyl oder gegebenenfalls durch Halogen, Alkyl, C₃₋₇-Cycloalkyl oder C₁₋₆-Alkoxy mono- oder gleich oder verschieden di-substituiertes Phenyl, einen 2-, 3- oder 4-Pyridinyl- oder einen 2-oder 3-Thienylrest bedeuten, wobei Alkyl geradkettiges Alkyl mit 1 bis 6 Kohlenstoffatomen oder verzweigtes Alkyl mit 3 bis 6 Kohlenstoffatomen bedeutet, sowie deren Salze und Säureadditionssalze, Tautomere und optische Isomere.

2. Die in Anspruch 1 definierten Verbindungen in enantiomerer Form.

3. Verbindungen gemäß Anspruch 1 oder 2, worin R¹ und R Phenyl oder gegebenenfalls durch 3- und/oder 4-Stellung substituiertes Phenyl, einen 4-pyridinyl- oder einen 3-Thienylrest bedeuten.

4. Verbindungen gemäß Anspruch 3, worin der Phenylrest durch Halogen oder Alkoxy substituiert ist.

5. Verbindungen gemäß Anspruch 1 oder 2, worin R¹ und R Phenyl sind.

6. (R,S)-4-(3-(1-Diphenylmethyl-azetidin-3-oxy)-2-hydroxypropylamino)-1H-indol-2-carbonitril gemäß Anspruch 1.

7. Verfahren zur Herstellung von Verbindungen der Formel I gemäß Anspruch 1,dadurch gekennzeichnet, daß man Verbindungen der Formel II, 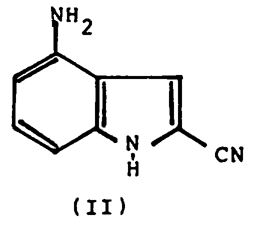 mit Verbindungen der Formel III 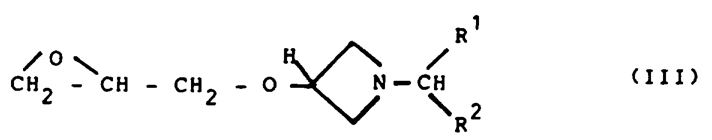 in der R¹ und R die oben angegebene Bedeutung haben, umsetzt.

8. Pharmazeutische Zubereitung, gekennzeichnet durch einen Gehalt an mindestens einer Verbindung der Formel I oder einem ihrer physiologisch unbedenklichen Salze.

9. Pharmazeutische Zubereitung, gekennzeichnet durch einen Gehalt an mindestens einer Verbindung gemäß Anspruch 5 oder 6 oder einem ihrer physiologisch unbedenklichen Salze.

10. Verbindungen der Formel I zur Bekämpfung von Krankheiten.

11. Verwendung von Verbindungen der Formel I zur Herstellung eines Arzneimittels.

## Patentansprüche (Patentansprüche für folgende(n) Vertragsstaat(en): ES)

1. Verfahren zur Herstellung von Indolylpropanolen der Formel I, 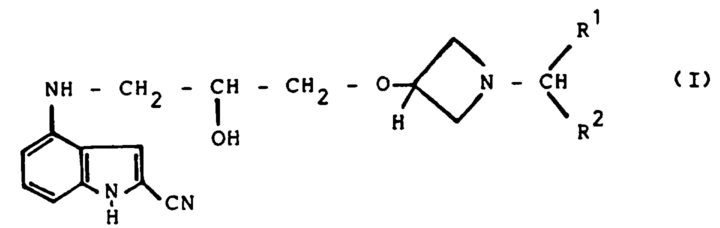 in der R¹ und R, die gleich oder verschieden sein können, jeweils Phenyl oder gegebenenfalls durch Halogen, Alkyl, C₃₋₇-Cycloalkyl oder C₁₋₆-Alkoxy mono- oder gleich oder verschieden di-substituiertes Phenyl, einen 2-, 3- oder 4-Pyridinyl- oder einen 2- oder 3-Thienylrest bedeuten, wobei Alkyl geradkettiges Alkyl mit 1 bis 6 Kohlenstoffatomen oder verzweigtes Alkyl mit 3 bis 6 Kohlenstoffatomen bedeutet, sowie deren Salzen und Säureadditionssalzen, Tautomeren und optischen Isomeren, dadurch gekennzeichnet, daß man Verbindungen der Formel II, 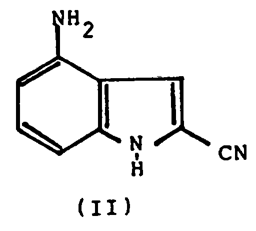 mit Verbindungen der Formel III 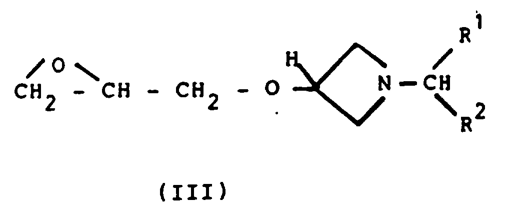 in der R¹ und R die oben angegebene Bedeutung haben, umsetzt und/oder erwünschtenfalls, ein erhaltenes Gemisch von diastereomeren Racematen in die entsprechenden diastereomeren Racemate oder optisch reinen Diastereomeren auftrennt, und/oder erwünschtenfalls, ein erhaltenes Racemat in die beiden optischen Antipoden auftrennt, und/oder erwünschtenfalls, eine erhaltene basische Verbindung der Formel I in ein Säureadditionssalz überführt.

2. Verfahren gemäß Anspruch 1, dadurch gekennzeichnet, daß man eine Verbindung, worin R¹ und R Phenyl sind, herstellt.

3. Verfahren gemäß Anspruch 1, dadurch gekennzeichnet, daß man (R,S)-4-(3-(1-Diphenylmethyl-azetidin-3-oxy)-2-hydroxypropyl-amino)-1H-indol-2-carbonitril herstellt.

## Claims (Claims for the following Contracting State(s): AT, BE, CH, DE, DK, FR, GB, IT, LI, NL, SE)

1. Substituted indolylpropanols of the Formula I 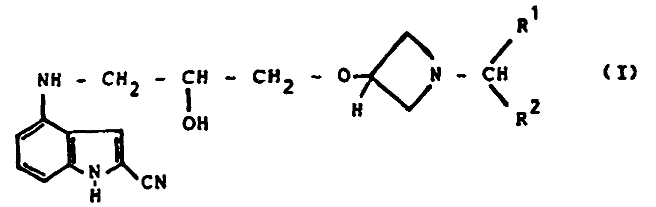 in which R¹ and R, which can be identical or different, are each phenyl or phenyl which is optionally monosubstituted or disubstituted in an identical or different manner, by halogen, alkyl, C₃₋₇-cycloalkyl and C₁₋₆-alkoxy, or a 2-, 3- or 4-pyridinyl or a 2- or 3-thienyl radical, where alkyl is straight-chain alkyl having 1 to 6 carbon atoms or branched alkyl having 3 to 6 carbon atoms, and salts and acid addition salts, tautomers and optical isomers thereof.

2. The compounds defined in Claim 1 in an enantiomeric form.

3. Compounds according to Claim 1 or 2, wherein R¹ and R are phenyl or phenyl which is optionally substituted in the 3- and/or 4-position, or a 4-pyridinyl or a 3-thienyl radical.

4. Compounds according to Claim 3, wherein the phenyl radical is substituted by halogen or alkoxy.

5. Compounds according to Claim 1 or 2, wherein R¹ and R are phenyl.

6. (R,S)-4-(3-(1-diphenylmethyl-azetidin-3-oxy)-2-hydroxypropylamino) -1H-indole-2-carbonitrile according to Claim 1.

7. Process for the preparation of compounds of the Formula I according to Claim 1, characterized in that compounds of the Formula II, 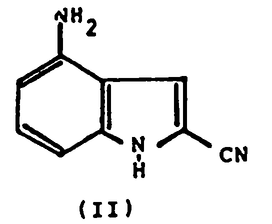 are reacted with compounds of the Formula III 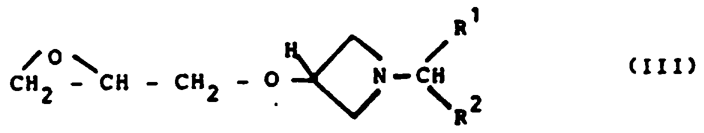 in which R¹ and R have the abovementioned meaning.

8. Pharmaceutical formulation, characterized by a content of at least one compound of the Formula I or one of its physiologically acceptable salts.

9. Pharmaceutical formulation, characterized by a content of at least one compound according to Claim 5 or 6 or one of its physiologically acceptable salts.

10. Compounds of the Formula I for combatting diseases.

11. Use of compounds of the Formula I for the preparation of a medicament.

## Claims (Claims for the following Contracting State(s): ES)

1. Process for the preparation of indolylpropanols of the Formula I 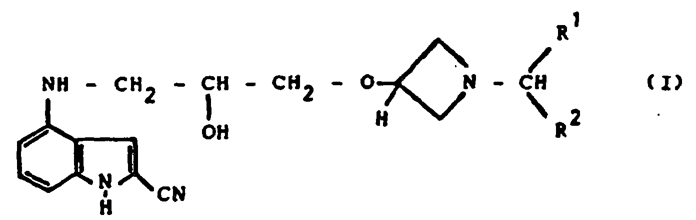 in which R¹ and R, which can be identical or different, are each phenyl or phenyl which is optionally monosubstituted or disubstituted in an identical or different manner, by halogen, alkyl, C₃₋₇-cycloalkyl and C₁₋₆-alkoxy, or a 2-, 3- or 4-pyridinyl or a 2- or 3-thienyl radical, where alkyl is straight-chain alkyl having 1 to 6 carbon atoms or branched alkyl having 3 to 6 carbon atoms, and salts and acid addition salts, tautomers and optical isomers thereof, characterized in that compounds of the Formula II 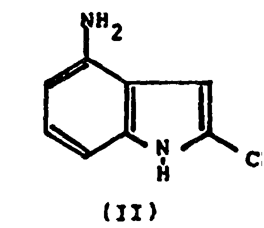 are reacted with compounds of the Formula III 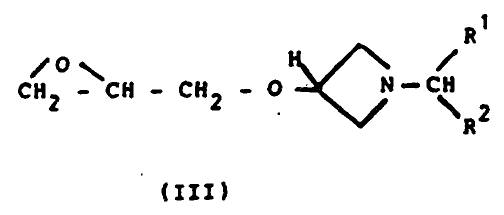 in which R¹ and R have the abovementioned meaning, and/or, if desired, a resulting mixture of diastereomeric racemates is separated into the corresponding diastereomeric racemates or optically pure diastereomers, and/or, if desired, a resulting racemate is separated into the two optical antipodes, and/or, if desired, a resulting basic compound of the Formula I is converted into an acid addition salt.

2. Process according to Claim 1, characterized in that a compound wherein R¹ and R are phenyl is prepared.

3. Process according to Claim 1, characterized in that (R,S)-4-(3-(1-diphenylmethyl-azetidin-3-oxy)-2-hydroxypropylamino)-1H-indole-2-carbonitrile is prepared.

## Revendications (Revendications pour l'(les) Etat(s) contractant(s) suivant(s): AT, BE, CH, DE, DK, FR, GB, IT, LI, NL, SE)

1. Indolylpropanols substitués répondant à la formule I 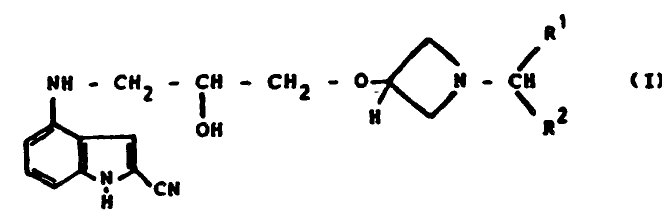 dans laquelle R¹ et R, qui peuvent être identiques ou différents, représentent respectivement un groupe phényle ou un groupe phényle éventuellement mono- ou disubstitué de manière identique ou différente par un atome d'halogène, par un groupe alkyle, par un groupe cycloalkyle en C₃-C₇ ou par un groupe alcoxy en C₁-C₆, un radical 2-, 3- ou 4-pyridinyle ou encore un radical 2- ou 3-thiényle; un groupe alkyle désignant un groupe alkyle à chaîne droite contenant de 1 à 6 atomes de carbone ou un groupe alkyle à chaîne ramifiée contenant de 3 à 6 atomes de carbone, ainsi que leurs sels et leurs sels d'addition d'acides, leurs tautomères et leurs isomères optiques.

2. Composés définis à la revendication 1, sous forme énantiomère.

3. Composés selon la revendication 1 ou 2, dans lesquels R¹ et R représentent un groupe phényle ou un groupe phényle éventuellement substitué en position 3 et/ou 4, un radical 4-pyridinyle ou un radical 3-thiényle.

4. Composés selon la revendication 3, dans lesquels le radical phényle est substitué par un atome d'halogène ou par un groupe alcoxy.

5. Composés selon la revendication 1 ou 2, dans lesquels R¹ et R représentent un groupe phényle.

6. (R,S)-4-(3-(1-diphénylméthyl-azétidin-3-oxy)-2-hydroxy-propylamino)-1H-indol-2-carbonitrile selon la revendication 1.

7. Procédé pour la préparation de composés répondant à la formule 1 selon la revendication 1, caractérisé en ce qu'on fait réagir des composés de formule II 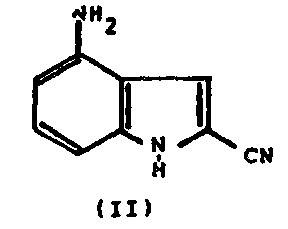 avec des composés de formule III 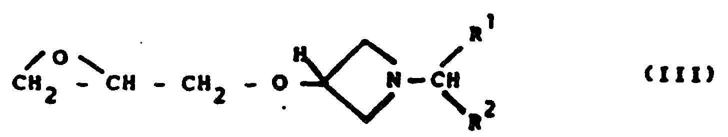 dans laquelle R¹ et R ont la signification indiquée ci-dessus.

8. Préparation pharmaceutique caractérisée par une teneur en au moins un composé de formule I ou en au moins un de ses sels physiologiquement acceptables.

9. Préparation pharmaceutique caractérisée par une teneur en au moins un composé selon la revendication 5 ou 6 ou encore en au moins un de ses sels physiologiquement acceptables.

10. Composés de formule I pour lutter contre des maladies.

11. Utilisation de composés de formule I pour la préparation d'un médicament.

## Revendications (Revendications pour l'(les) Etat(s) contractant(s) suivant(s): ES)

1. Procédé pour la préparation d'indolylpropanols de formule I 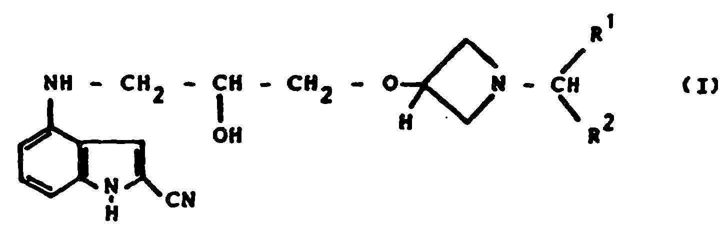 dans laquelle R¹ et R, qui peuvent être identiques ou différents, représentent respectivement un groupe phényle ou un groupe phényle éventuellement mono- ou disubstitué de manière identique ou différente par un atome d'halogène, par un groupe alkyle, par un groupe cycloalkyle en C₃-C₇ ou par un groupe alcoxy en C₁-C₆, un radical 2-, 3- ou 4-pyridinyle ou encore un radical 2- ou 3-thiényle; un groupe alkyle désignant un groupe alkyle à chaîne droite contenant de 1 à 6 atomes de carbone ou un groupe alkyle à chaîne ramifiée contenant de 3 à 6 atomes de carbone, ainsi que de leurs sels et de leurs sels d'addition d'acides, de leurs tautomères et de leurs isomères optiques, caractérisé en ce qu'on fait réagir des composés de formule II 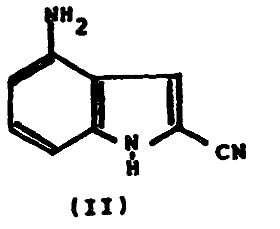 avec des composés de formule III 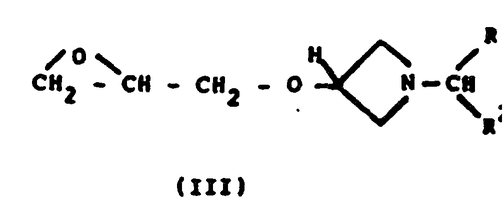 dans laquelle R¹ et R ont la signification indiquée ci-dessus, et/ou, si on le souhaite, on dédouble un mélange obtenu de racémates diastéréo-isomères en racémates diastéréo-isomères correspondants ou en diastéréo-isomères optiquement purs, et/ou, si on le souhaite, on dédouble un racémate obtenu en deux antipodes optiques, et/ou, si on le souhaite, on transforme un composé basique obtenu de formule I en un sel d'addition d'acides.

2. Procédé selon la revendication 1, caractérisé en ce qu'on prépare un composé dans lequel R¹ et R représentent un groupe phényle.

3. Procédé selon la revendication 1, caractérisé en ce qu'on prépare le (R,S)-4-(3-(1-diphénylméthyl-azétidin-3-oxy)-2-hydroxy-propylamino)-1H-indol-2-carbonitrile.
